# EUROPEAN PATENT APPLICATION

(11) **EP 0 872 814 A1**
(43) Date of publication of application: **21.10.1998**
(21) Application number: 97302580.2
(22) Date of filing: 15.04.1997
(51) Int. Cl.: G07C 9/00, A61B 3/12

(54) **Optical apparatus**

(71) Applicant: BRITISH TELECOMMUNICATIONS public limited company, London EC1A 7AJ (GB)
(72) Inventor: The designation of the inventor has not yet been filed
(74) Representative: Lloyd, Barry George William

(57) **Abstract**

An optical apparatus which records light (B) reflected from one or more of a user's facial features is disclosed. Whilst the image is captured, visible light (A) from a display (170) or other scene to be viewed by the user passes to the user's eye (110). Prior apparatuses of this type have been arranged such that a significant proportion of the light reflected from the user's face is not incident on an image capture device inside the apparatus. The embodiment described is a portable iris pattern capture device (12). In the embodiment near-infra red light (B) is directed towards the user's eye (110), from where it is reflected back into the apparatus. Visible light (A) from an LCD display (170) mounted at the rear of the apparatus travels out to the user's eye (110) from the apparatus. A hot mirror (145) inside the apparatus directs the near infra-red light (B) reflected from the user's eye to a camera unit (135), and does not significantly attenuate the visible light (A) emanating from the display (170).

## Description

The present invention relates to an optical apparatus. It has particular utility in relation to apparatuses which are required to capture an image of a user's eye and display an image to the user simultaneously. Such an apparatus might be used in an authentication device which captures an image of an iris of a user.

Apparatuses for capturing an image of a user's eye at the same time as providing a display for viewing by the user's eye are known. For example, IriScan Inc. market a System 2000EAC™ authentication apparatus. Another example is the pupillometer apparatus disclosed in international patent application WO 92/05736.

The former apparatus comprises an iris pattern capture unit and an identification code generation unit. The iris pattern capture unit has a housing having two apertures. An infrared illumination source is arranged to project visible and infra-red light through the first aperture to illuminate a user's eye. A camera, installed within the apparatus is arranged such that its field of view is through the second aperture. In using the apparatus the user looks through the second aperture at a display which reproduces the image seen by the camera. At the same time, light from the illumination source is reflected from the user's eye through the second aperture towards the camera. The display enables the user to position his eye such that it is in focus and located in the centre of the field of view of the camera.

In practice it is found that the user must position his or her eye within a narrow range of distances from the device in order for a successful capture of an image of the user's eye to be achieved.

This is one of the problems addressed by the present invention, which provides an optical apparatus comprising:
a user image capture apparatus operable to capture a user image of one or more facial features of a user responsive to the incidence of light reflected from the user; and
an optical device;
   wherein, in use, visible light travels along a first optical path via the optical device to the user's eye to provide a visible image to be viewed by the user and user light reflected from the user travels along a second optical path via the optical device to said image capture apparatus;
   wherein said optical device is arranged to interact with at least one of said user light and said visible light such that substantially all user light of a predetermined type travels further along said second optical path towards said image capture apparatus, and a significant proportion of said visible light travels further along said first optical path towards the user.

The terms 'optical' and 'light' as used herein relate to electromagnetic radiation in the infra-red, visible and ultra-violet portions of the electromagnetic spectrum, i.e. having wavelengths in the range 10nm to 1mm.

Because most of the user light of the predetermined type arrives at the image capture apparatus, the present invention achieves a number of advantages.

Firstly, since more light is received at the image capture apparatus, the aperture of the optical arrangement associated with it can be reduced. This has the result that the depth of focus of the image capture apparatus is increased. Thus, the constraints placed on where the user must position his face in relation to the apparatus in order to provide a satisfactorily focused image are relaxed.

Secondly, the time of exposure may be reduced, thereby reducing the need for the user to remain still whilst the user image is captured.

Thirdly, the present invention also allows a satisfactory user image to be captured in situations where less light to which the user image capturing apparatus is responsive is available. This might prove beneficial in relation to apparatuses which illuminate the user's eye since safety considerations limit the light that can directed towards the eye. It might also prove useful in reducing power requirements in apparatuses which illuminate the user, this being a particular problem in relation to portable equipment powered by batteries.

Those skilled in the art will realise that the above advantages could be realised individually or in combination. Generally, providing more light to an image capture apparatus improves the quality of the resultant image.

Any increase in the proportion of said user light of a predetermined type which is incident upon the image capture apparatus (in comparison to known apparatuses) leads to some improvement in performance. However, it is preferred that more than 75% of the user light of a predetermined type travels further along the optical path towards the image capture apparatus.

In addition to the above, allowing the visible light to pass substantially unattenuated towards the user increases the apparent brightness of the display. This means that the amount of power consumed by the apparatus is reduced; a particularly useful feature in portable apparatuses. It is preferred that more than 75% of the visible light travels further along the optical path towards the user.

In some cases, the visible image seen by the user might simply be a view through the apparatus. In others, a display means provides the visible image or overlays a partial display on that already present to provide the visible image. This has the advantage that information can be provided to the user at the time he uses the apparatus. Looked at another way, the user image capture unit can be operated whilst the user is viewing information on the display.

One example of the use of a display involves the provision of an display which shows the user image being received by the user image capture apparatus. This is advantageous since it allows the user to move his head in order to improve the quality of the image captured.

In preferred embodiments the visible light directed towards the user and the light directed towards the user image capture apparatus have different spectra. This does not mean that the light directed towards the user image capture apparatus necessarily lies outside the visible region of the electromagnetic spectrum. For example, the display might be created using red, blue and green LEDs which emit light in narrow spectral bands, the user image capture apparatus receiving visible light which falls outside those bands but within the visible portion of the electromagnetic spectrum. Such an arrangement, might, for example be used in an eye-to-eye video phone (where the field of view of the camera at each site is straight out from the display, whereby eye contact can be made by the parties during conversation).

In advantageous versions of those embodiments the optical device comprises a wavelength selective reflector effective to reflect either said visible light or said light of a different spectrum, and to allow the passage of the other. Such reflectors are economical and their use allows the components of the apparatus to be conveniently located. The selectivity can, for example, be achieved using metallic coatings or coatings of dielectric material(s).

Since the user light is incident upon the user's face, the apparatus is more comfortable for the user if the predetermined type of light substantially consists of non-visible light.

Preferably, the predetermined type of light substantially consists of near infra-red light (i.e. having a wavelength between 700nm and 3µm). This is preferable because only unusually hot objects will emit significant amounts of light in that region of the electromagnetic spectrum, and thereby cause distortion of the image.

Further advantages are obtained when the optical device comprises a 'hot mirror'. A hot mirror is a device which is operable to reflect substantially all infra-red light whilst allowing the passage of visible light. It will be realised by those skilled in the art that a 'cold mirror' (which reflects substantially all visible light and allows the passage of infrared light) could equally be used.

Advantageously, the apparatus further comprises an illumination source operable to illuminate the user with light of said predetermined type. The advantage here is that both the spectrum and direction of the light used in forming the image can be controlled.

Particularly useful embodiments arise when the apparatus is designed to capture an image of one of the user's irises. The light impinging on the user's eye can be non-visible, making the apparatus comfortable in use. Furthermore, the intensity of the user light may be reduced owing to the increased amount of light incident on the image capture apparatus. Again, this makes the apparatus more comfortable in use.

Specific embodiments of the present invention will now be described, by way of example only, with reference to the accompanying figures, in which:
Figure 1 illustrates a personal computer arranged for remote access to a shared computer;
Figure 2 is a schematic representation of an iris imaging apparatus;
Figure 3 is a schematic diagram which illustrates one possible hardware architecture for the imaging apparatus;
Figure 4 is a flow chart of an image capturing and encryption process; and
Figures 5a and 5b are diagrams which illustrate the display provided on the user correctly and incorrectly aligning his eye respectively.

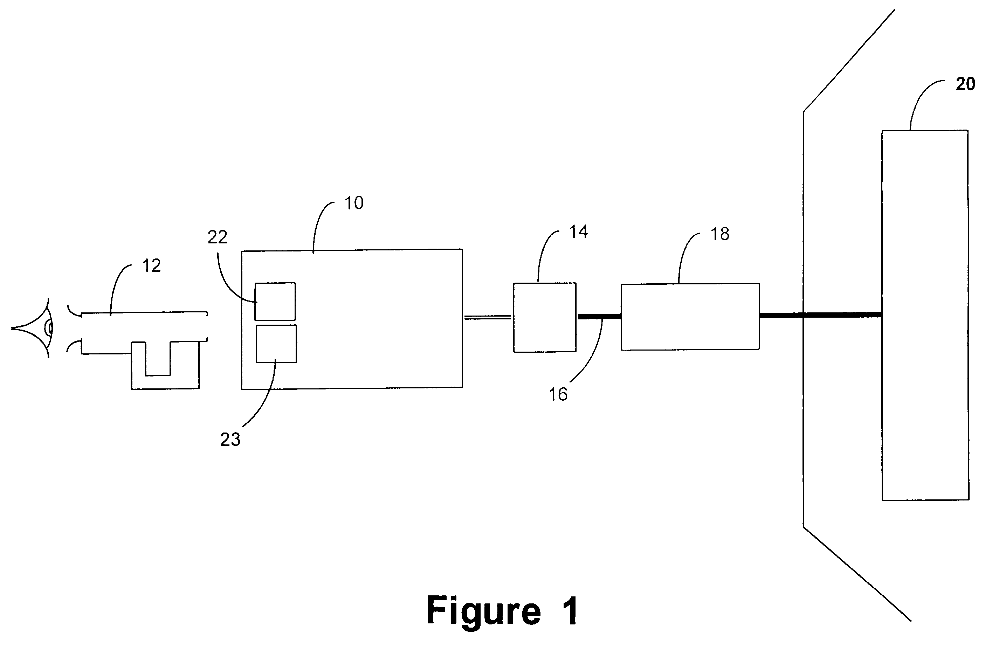

Figure 1 shows a user's personal computer (PC) 10 which, in addition to normal input/output devices and associated interfaces, has an infra-red receiver 22 and transmitter 23 and an infra-red signal interface card (not shown). These additional components enable communication of data between the user's PC 10 and an image capture apparatus 12.

The user's PC 10 is connected via a modem 14 and a telecommunications line 16 to an intermediate routing computer 18, which routes signals from the user's PC 10 to a server 20. The server 20 may, for example, belong to a corporation and have storage means containing files which are of significant value to that corporation. However, where the user of the PC 10 is an employee of that corporation, then the provision of a communications link between the PC 10 and the server 20 has the benefit of allowing that employee to work from home.

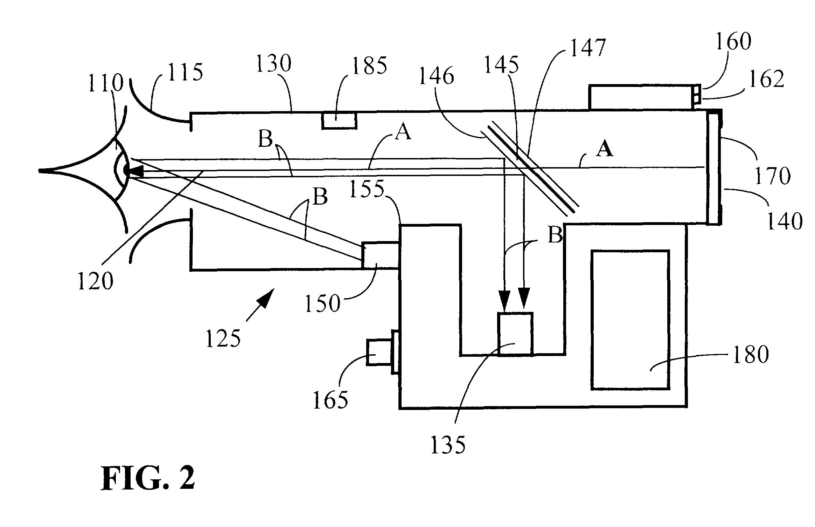

Figure 2 illustrates the image capture apparatus in more detail. The apparatus has a housing 125 which is generally T-shaped having an elongate horizontal barrel section 130 and a handle section which depends downwardly from the barrel 130 from a position intermediate its ends. The handle section is substantially hollow and is closed at its lowermost end. A charge coupled device (CCD) camera 135 is located at the base of the interior of the handle section. The camera is a black and white camera approximately equally sensitive to visible and near infra-red light. Connections (not shown) are provided from the output of the CCD camera 135 to a rearwardly facing liquid-crystal display (LCD) 170 mounted on the inside of the front end of the barrel 130. The handle portion also includes appropriate electronic circuitry 180 contained in the housing (described in more detail in relation to Figure 2).

A window 120 is formed in the rearward end of the barrel 130. An eye-cup 115 is attached to the rearward end of the barrel and surrounds the window 120. The eye-cup 115 acts both as a means of minimising the amount of ambient light entering the apparatus and as the means for aligning the user's eye with the window 120.

A 'hot mirror' 145 (for example, as sold under catalogue number 35-6681 by Ealing Optics of Greylaine Rd, Watford, U.K.) is located directly above the CCD camera 135. The mirror 145 slopes downwardly and forwardly at 45° to the longitudinal axis of the barrel 130. The mirror is formed from a glass slide having a coating of dielectric materials on its underside. The other side of the glass slide is coated with an anti-reflective coating 147. The coating 146 of dielectric material is effective to reflect approximately 80% of near infra-red light which falls upon it and to allow the passage of approximately 90% of visible light which falls upon it.

A near infra-red light-emitting diode (LED) 150 is located on the inside of the barrel 130 between the mirror 145 and the eye-cup 115 and is operable to illuminate the user's eye. The source is mounted on the rearward facing side of a screen 155 to prevent light travelling directly from the source 150, via the mirror 145, to the camera 135. The barrel 130 also carries an internally-mounted optical indicator 185 comprising a red and a green LED positioned so as to be in the field of view of the user.

An infra-red (IR) transmitter 160 and receiver 162 are located on the front of the housing 125, in line with the longitudinal axis of the barrel 130, and a trigger button 165 is included on the handle portion operable by the user to control when the image is captured and/or transmitted to the user's PC 10.

The overall size of the apparatus depends mainly on the size of the opening for the eye 120 and on the level of comfort and ease of use required by a user of the apparatus. The hardware for the apparatus is designed onto a single ASIC chip, the size of which is not a limiting factor to the size of the apparatus. Also, known CCD cameras can have dimensions in the order of millimetres (or tens of millimetres if the size of the printed circuit board mounting is taken into consideration) and are not a limiting factor of the apparatus size.

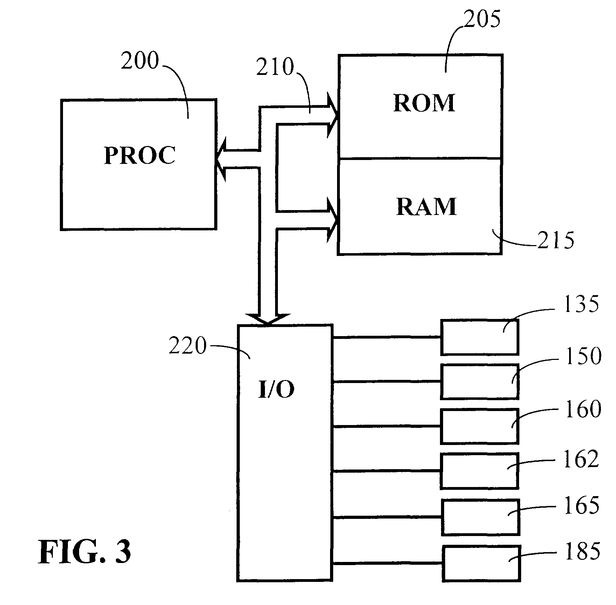

Figure 3 shows one possible hardware architecture arrangement for the apparatus. As already stated, the processing hardware is preferably engineered onto a single application specific integrated circuit (ASIC). The apparatus is controlled by a processor 200 which runs software held in ROM 205. The processor 200 is connected via a bus 210 to the ROM 205, a block of RAM 215 and an input/output (I/O) controller 220. The RAM is large enough to hold at least one captured image of an eye. The I/O controller 220 is connected by appropriate circuitry and drivers (not shown) to the IR transmitter 160 and receiver 162, the CCD camera 135, the trigger 165, the near infra-red LED 150 and the optical indicator 185. The whole apparatus is powered by a suitable battery (not shown).

The processor 200 is sensitive to signals received from the trigger 165, the IR receiver 162 and the CCD camera 135. Also, the processor controls the IR transmitter 160, the near infra-red LED 150, the CCD camera operation and the optical indicator 185.

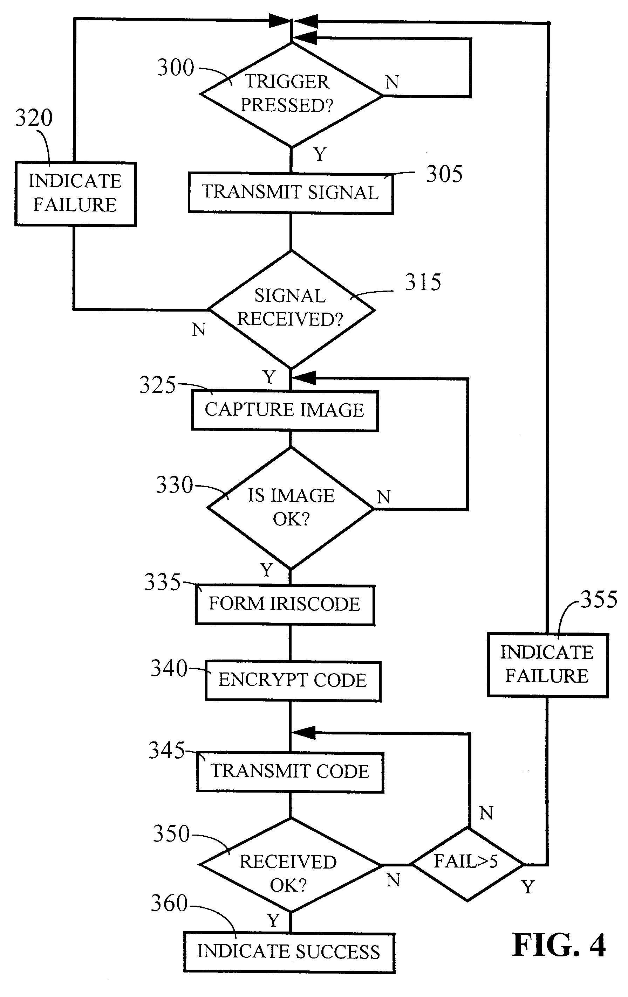

The flow diagram in Figure 4 illustrates one possible process for the image capturing, processing and transmitting aspects of a user validation system. This procedure includes encryption to enhance the level of security. The encryption system uses a 'public key' to encipher data and a private key (known only to the recipient of the enciphered data) to decipher the data.

In step 300, the imaging apparatus 12 is in a state where a trigger depression is awaited to start the process. The user brings the imaging apparatus 12 to his eye and presses the trigger causing the generation a signal which is received by the processor. The processor then controls the IR transmitter 160 to send a signal, in step 305, to the user's PC 10 to initiate communications. In response, the user's PC 10 sends a return message to the imaging apparatus 12.

If the return message is not received by the imaging apparatus 12 in step 315, for example as a result of the user's PC 10 not receiving the first signal, the red LED of the optical indicator lights in step 320 to indicate failure and inform the user to re-start the process by pressing the trigger 165 again.

If the return message is received in step 315, the signal from the user's PC 10 includes a selection of which public encryption key and which iris code format the imaging apparatus 12 must use for successful transmission. A plurality of public encryption keys and a plurality of iris code algorithms from which the selection can be made are stored in the RAM (or the ROM) in the imaging apparatus 12. The user's PC 10 also transmits a date and time stamp to the imaging apparatus 12.

The information in the return signal, transmitted by the user's PC 10, is stored in the RAM in the imaging apparatus 12 for subsequent access.

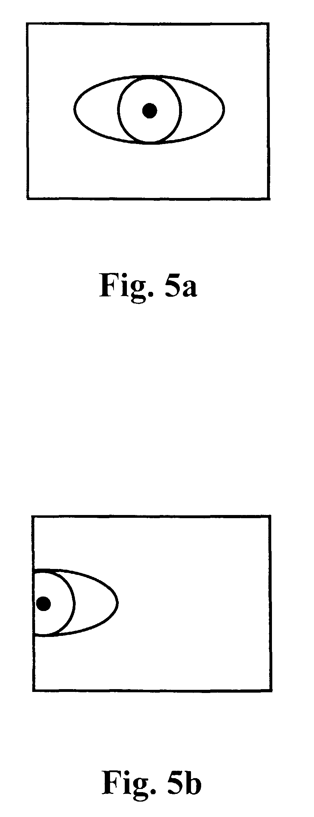

Next, in step 325, the processing means signals to the camera that one or more images should be captured. Image data captured by the CCD camera 135 is sent over the above-mentioned connection to the display 170 such that the display 170 echoes the image currently being received by the CCD camera 135. Light from the display travels along the path A through the hot mirror 145 to the user's eye 110. The light is only slightly attenuated by the mirror (approximately 90% of the visible light is transmitted. If the user's eye is correctly aligned with the window 120 then he or she sees a display like the one illustrated in Figure 5a. If, however, the user's eye is not correctly aligned then he or she sees a display like that illustrated in Figure 5b. The user can then change the position of his or her eye relative to the window accordingly.

The near infra-red LED 150 illuminates the user's eye 110, the light from the LED following the path B, i.e. it travels from the infra-red LED 150 to the user's iris where it is reflected towards the hot mirror 145. On reaching the hot mirror 80% of the near infra-red light is reflected by the surface coating 146 towards the CCD camera 135.

The images which are captured are stored in the RAM 215. In step 330, the processing means determines if the stored image, or which image, is suitable for encoding. If none of the one or more images is suitable, the processor signals to the camera to re-capture the image(s).

The image capturing step includes control of the near infra-red LED 150. The near infra-red LED 150 is connected in a control loop whereby the processor 200 can vary the light intensity of the source 150 depending on, for example, the colour of the user's iris: a light blue iris reflects far more light and needs less illumination than a dark brown iris. Several sequentially captured images, similar to a video sequence, might be required for the processor and software to determine the optimum illumination for the eye before a suitable image, or suitable images, is/are obtained.

It is suggested that pulsing the near infra-red LED 150 is more desirable than using a continuous source, although the image capture would need to be synchronised with a pulse of light to ensure suitable illumination. Pulsing light has the advantage that the user's eye is exposed, on average, to less optical radiation. Also, a pulsed source uses less energy.

Capturing multiple images can also overcome problems such as, for example, the user blinking at the point when one image is captured. Known digital signal processing techniques can be used to establish which image is the best and to reject unsuitable images.

When a suitable image is obtained, the image data is retrieved from the RAM 215 and is processed to form an iris code, in step 335, using the iris code generating algorithm selected by the user's PC 10 in step 315. An example algorithm is that described in US patent 5,291,560. The resulting iris code is stored in the RAM.

The processor 200 then encrypts the iris code, in step 340, using the selected public key, along with the date and time stamp provided by the user's PC 10 in step 315. The resulting data is stored in RAM 215. The coded and encrypted data is then transmitted to the user's PC 10 by the IR transmitter 160 in step 345.

It is feasible that the image capture, processing and encryption steps are completed without any intervening steps of storing data in RAM, that is to say processing is done "on-the-fly", to greatly increase the speed of operation of the apparatus. However, such processing would require more expensive and more complex electronics.

Finally, if the data is received successfully by the user's PC 10, the user's PC 10 returns a 'success' signal to the imaging apparatus 12 in step 350. The processing means, in response, causes the green LED of the optical indicator 185 to light to indicate to the user that the procedure has been successful in step 360. Repeated failure to transmit the data, for example after five attempts, causes the red LED of the optical indicator 185 to light in step 355 and results in the user needing to re-start the whole procedure.

A simpler process than that described above involves the imaging apparatus 12 dictating which of the plurality of public encryption keys to use. The selection can be made using a pseudo-random number generator in the imaging apparatus 12. If each public key has an index reference, the respective reference can be included, obviously in non-encrypted form, with the encrypted data to indicate to the user's PC 10 which public key has been used and, thus, which private key should be used for de-encryption. An extension to this arrangement is that a new set of public keys is down-loaded to the imaging apparatus 12, from the user's PC 10, each time a successful transaction occurs. Other, further encryption possibilities will be apparent to the skilled person.

In practice, the near infra-red LED 150 is controlled by the processor 200 (via suitable electrical circuitry which is not shown). The processor 200 controls when the infra-red LED 150 lights up to illuminate the eye, either in response to its own controlling software or in response to signals received from the user's PC 10. The processor also determines when, and under which lighting conditions, the image capturing process occurs.

As an alternative to the hot mirror used in the above embodiment, a cold-mirror may be used. The cold mirror has the same position and orientation as the hot mirror but has a different coating on its underside. The coating would be effective to reflect most visible light whilst allowing the passage of near infra-red light. The other alteration which is made in this alternative embodiment is to swap the CCD camera and the LCD display about.

It will be seen how the above described embodiments provide an optical apparatus which directs more user light towards the camera than known apparatuses. In the above embodiments this leads to the advantage that the near infra-red LED 150 need not be as bright as it would otherwise be. This means that the battery in the apparatus will last for longer and that less light will be directed towards the user's eye, making the apparatus safer to use. If the brightness of the near infra-red LED 150 is maintained at a level consistent with known apparatuses then a better image of the user's eye will be obtained. The increased light falling on the CCD camera will result in the effect of electronic noise being decreased in comparison to known apparatuses.

Although the above embodiment relates to a portable apparatus, the invention may also be applied in fixed apparatuses. For example, the optical arrangement disclosed above could be incorporated into the known System 2000EAC™ authentication apparatus. If this were done, the aperture of the optics associated with the camera in that apparatus could be stopped down, thereby increasing the depth of focus of the camera and making the apparatus easier to use.

In addition, problems associated with reflections from the user's eye of light sources other than the unit's source would be reduced. The unit's source is positioned so as to minimise reflections from the cornea of the user which tend to 'wash out' the user image. However, the human cornea is highly reflective and strongly reflects light from other sources. Usually these sources cause unwanted reflections from the cornea thereby spoiling the performance of the apparatus. By placing the above described optical system into the System 2000EAC™ apparatus the effect of visible reflections can be significantly reduced. Thus the incorporation of the above-described optical arrangement provides enhanced performance in the presence of any extraneous light sources that have some visible light within their output.

An optical arrangement similar to that described above may also be incorporated in an automated teller machine (ATM). The ATM might then require the presentation of an iris pattern matching that of an account holder before allowing the withdrawal of money from that account. Again, the present invention would be particularly useful because the increased depth of focus of the apparatus would make the ATM easier to use.

## Claims

1. An optical apparatus comprising:
a user image capture apparatus operable to capture a user image of one or more facial features of a user responsive to the incidence of light reflected from the user; and
an optical device;
wherein, in use, visible light travels along a first optical path via the optical device to the user's eye to provide a visible image to be viewed by the user and user light reflected from the user travels along a second optical path via the optical device to said image capture apparatus;
wherein said optical device is arranged to interact with at least one of said user light and said visible light such that substantially all user light of a predetermined type travels further along said second optical path towards said image capture apparatus, and a significant proportion of said visible light travels further along said first optical path towards the user.

2. An apparatus according to claim 1 wherein said visible light travels substantially unattenuated along said first optical path towards the user.

3. An apparatus according to claim 1 or 2 further comprising a display means operable to provide at least part of said visible image.

4. An apparatus according to claim 3 wherein said display means is operable, in use, to display the user image captured by the image capture apparatus.

5. An apparatus according to any preceding claim wherein said predetermined type of light differs in spectrum from said visible light.

6. An apparatus according to claim 5 wherein said optical device comprises a wavelength selective reflector effective to reflect either said visible light or said light of a different spectrum, and to allow the passage of the other.

7. An apparatus according to any preceding claim wherein said predetermined type of light substantially consists of non-visible light.

8. An apparatus according to claim 7 wherein said type of light substantially consists of near infra-red light.

9. An apparatus according to claim 8 wherein said optical device comprises a hot mirror.

10. An apparatus according to any preceding claim further comprising an illumination source operable to illuminate the user with light of said predetermined type.

11. An apparatus according to any preceding claim wherein said one or more facial features comprise an iris pattern of the user.

12. An image capture apparatus substantially as hereinbefore described with reference to and as illustrated in Figures 1 to 3.
